# EUROPEAN PATENT APPLICATION

(11) **EP 4 846 923 A1**
(43) Date of publication of application: **02.12.2026**
(21) Application number: 25747958.4
(22) Date of filing: 26.01.2025
(51) Int. Cl.: A61B 3/032, A61B 3/028

(54) **OPHTHALMIC OPTICAL INSTRUMENT AND OPTICAL MEASUREMENT SYSTEM**

(30) Priority: 30.01.2024 CN 202410130018
(71) Applicant: Affiliated Eye Hospital of Wenzhou Medical University, Wenzhou, Zhejiang 325027 (CN)
(72) Inventor: WANG, Guangji, Wenzhou, Zhejiang 325027 (CN); CHEN, Jie, Wenzhou, Zhejiang 325027 (CN); QU, Jia, Wenzhou, Zhejiang 325027 (CN); LV, Fan, Wenzhou, Zhejiang 325027 (CN); WANG, Yong, Wenzhou, Zhejiang 325027 (CN)
(74) Representative: Gulde & Partner
(86) International application number: PCT/CN2025/075242
(87) International publication number: WO 2025/162394

(57) **Abstract**

Provided are an ophthalmic optical instrument and an optical measurement system. The ophthalmic optical instrument comprises a visual target element, a first lens, a second lens, and a third lens. The first lens and the visual target element are arranged at intervals, and light emitted from the visual target element enters the first lens and exits as parallel light. The second lens is located on one side of the first lens away from the visual target element, and the described parallel light is converged to form an image after passing through the second lens. The third lens is located on one side of the second lens away from the first lens. The ophthalmic optical instrument further comprises a control element connected to the second lens and configured to control the movement of the second lens along its principal optical axis, such that the image formed by the second lens moves to at least one side of the third lens. The second lens in the ophthalmic optical instrument can move along its principal optical axis under the control of the control element, allowing the image formed by the second lens to move accordingly. Therefore, the movement range of the image formed by the second lens is relatively large, thereby improving the measurement range.

## Description

The present application claims priority to Chinese Patent Application No. 2024101300187 filed on January 30, 2024, the entire contents of which are incorporated herein by reference as part of the present application.

### TECHNICAL FIELD

At least one embodiment of the present disclosure relates to an ophthalmic optical instrument and an optical measurement system.

### BACKGROUND

In modern times, with the development and progress of science and technology, modern life is closely related to electronic products. As people's dependence on electronic products continues to increase, the incidence of myopia keeps rising. Generally, the occurrence of myopia and presbyopia is closely related to the visual function (i.e., visual acuity) and accommodative capacity associated with near-distance work, study, and entertainment. In some studies, the probability that adolescents suffer from myopia is as high as 90%. Meanwhile, along with the phenomena of earlier onset of presbyopia and the aging of the population, about 1 billion people worldwide are affected by presbyopia.

### SUMMARY

At least one embodiment of the present disclosure provides an ophthalmic optical instrument and an optical measurement system.

At least one embodiment of the present disclosure provides an ophthalmic optical instrument, including: a visual target element, a first lens, a second lens and a third lens; the first lens is spaced apart from the visual target element, light emitted from the visual target element enters the first lens and exits as parallel light, the second lens is located on a side of the first lens away from the visual target element, the parallel light is converged to form an image after passing through the second lens; and the third lens is located on a side of the second lens away from the first lens, the ophthalmic optical instrument further includes a control element, the control element is connected to the second lens and configured to control movement of the second lens along its principal optical axis, so that an image formed by the second lens moves to at least one side of the third lens.

For example, according to the ophthalmic optical instrument provided by at least one embodiment of the present disclosure, the first lens is a collimator lens, the second lens includes a first positive lens, the third lens includes a second positive lens, and the control element is configured to control the second lens to move between the first lens and the third lens, with an image plane of the second lens being located on a side of the second lens away from the visual target element.

For example, according to the ophthalmic optical instrument provided by at least one embodiment of the present disclosure, a preset position is defined between the first lens and the third lens, and in response to that the second lens moves between the preset position and the third lens, the image plane of the second lens is located on a side of the third lens away from the visual target element.

For example, according to the ophthalmic optical instrument provided by at least one embodiment of the present disclosure, a focal length of the third lens is greater than a focal length of the second lens.

For example, according to the ophthalmic optical instrument provided by at least one embodiment of the present disclosure, the ophthalmic optical instrument further includes a lens barrel including a first end and a second end opposite to each other, the third lens is connected to the first end, and a central axis of the lens barrel coincides with a principal optical axis of the third lens, a distance between the first end and the second end is not greater than a focal length of the third lens.

For example, according to the ophthalmic optical instrument provided by at least one embodiment of the present disclosure, an entrance pupil center of an examinee's eye coincides with a back focal point of the third lens.

For example, according to the ophthalmic optical instrument provided by at least one embodiment of the present disclosure, a focal length of the first lens is greater than a focal length of the second lens.

For example, according to the ophthalmic optical instrument provided by at least one embodiment of the present disclosure, the focal length of the second lens is in a range from 1/5 to 4/5 of the focal length of the first lens.

For example, according to the ophthalmic optical instrument provided by at least one embodiment of the present disclosure, a distance between the first lens and the third lens is greater than a sum of the focal length of the second lens and the focal length of the third lens.

For example, according to the ophthalmic optical instrument provided by at least one embodiment of the present disclosure, the visual target element is located at a front focal point of the first lens.

For example, according to the ophthalmic optical instrument provided by at least one embodiment of the present disclosure, the ophthalmic optical instrument further includes a beam splitter and an automatic detection element, the beam splitter is located in the lens barrel, a principal optical axis of the beam splitter forms an inclination angle with a focal plane of the third lens, the inclination angle is in a range from 20 degrees to 50 degrees; the automatic detection element is configured to receive light from the beam splitter so as to detect a diopter of the examinee's eye.

For example, according to the ophthalmic optical instrument provided by at least one embodiment of the present disclosure, the visual target element includes an electronic display panel configured to display a target, the target includes at least one of a Chinese character, a numeral, and an English letter, the Chinese character includes at least one typeface.

For example, according to the ophthalmic optical instrument provided by at least one embodiment of the present disclosure, a shape and a size of the target are variable.

For example, according to the ophthalmic optical instrument provided by at least one embodiment of the present disclosure, the electronic display panel is further configured to provide a voice prompt.

For example, according to the ophthalmic optical instrument provided by at least one embodiment of the present disclosure, the target includes a plurality of sub-targets arranged as M sub-target rows, where M is a positive integer greater than 1, and along an arrangement direction of the M sub-target rows, a height of an i-th sub-target row, a distance between the i-th sub-target row and an (i+1)-th sub-target row, and a height of the (i+1)-th sub-target row are distributed in a geometric progression, where i is a positive integer less than or equal to M-1.

For example, according to the ophthalmic optical instrument provided by at least one embodiment of the present disclosure, the ophthalmic optical instrument further includes: a body and a mounting frame, the body is tubular, the first lens, the second lens, and the third lens are all located in the body, the body includes two ends opposite with each other, the light emitted from the visual target element enters the body through an end of the body close to the first lens, at least part of the lens barrel extends into the body from another end of the body, and the body is rotatably connected to the mounting frame such that an angle of the body relative to a setup plane of the mounting frame is adjustable.

For example, according to the ophthalmic optical instrument provided by at least one embodiment of the present disclosure, an outer diameter of the lens barrel is smaller than an inner diameter of the body.

At least one embodiment of the present disclosure further provides an optical measurement system, which includes the ophthalmic optical instrument according to any one of the above embodiments.

### BRIEF DESCRIPTION OF DRAWINGS

To describe the technical solutions of the embodiments of the present disclosure more clearly, the drawings of the embodiments will be briefly described below. Obviously, the drawings described below merely relate to some embodiments of the present disclosure, rather than limiting the present disclosure.
Fig. 1 is a schematic structural diagram of an ophthalmic optical instrument provided by at least one embodiment of the present disclosure.
Fig. 2 is a schematic diagram of a target provided by at least one embodiment of the present disclosure.
Fig. 3 is an optical path diagram in which an image formed by the second lens is located between the second lens and the third lens according to an embodiment of the present disclosure.
Fig. 4 is an optical path diagram in which an image formed by the second lens is located on a side of the third lens away from the second lens according to an embodiment of the present disclosure.
Fig. 5 is an optical path diagram of the ophthalmic optical instrument provided by an embodiment of the present disclosure in response to measuring a hyperopic eye.
Fig. 6 is an optical path diagram of the ophthalmic optical instrument provided by an embodiment of the present disclosure in response to measuring a myopic eye.

### DETAILED DESCRIPTION OF EMBODIMENTS

To make the objectives, technical solutions, and advantages of the embodiments of the present disclosure clearer, the technical solutions of the embodiments of the present disclosure will be clearly and completely described below with reference to the drawings of the embodiments of the present disclosure. Obviously, the described embodiments are part of, rather than all of, the embodiments of the present disclosure. All other embodiments obtained by a person of ordinary skill in the art on the basis of the described embodiments without creative effort shall fall within the protection scope of the present disclosure.

Unless otherwise defined, the technical or scientific terms used in the present disclosure shall have their ordinary meanings as understood by a person having ordinary skill in the art to which the present disclosure pertains. The words "first," "second," and similar words used in the present disclosure do not denote any order, quantity, or importance, but are merely used to distinguish different constituent parts. Words such as "comprising" or "including" mean that an element or object preceding such a word encompasses the elements or objects listed after it and their equivalents, without excluding other elements or objects.

Visual acuity refers to the ability of the retina to resolve an image. The quality of visual acuity is determined by the resolving power of the retina for an image. Generally, visual acuity examination includes examination of near visual acuity and distance visual acuity; for example, a measurement distance may be 0.25 meter or 5 meters. In some visual acuity examination processes, the visual acuity of an examinee's eye may be measured in response to that the retina is defocused, rather than in response to that a target is imaged clearly on the retina (i.e., in response to that the target is conjugate with the retina), so the examination result is prone to errors. In addition, some common visual acuity examination methods, such as the pinhole visual acuity measurement method, are used to examine potential best corrected visual acuity. From the perspective of geometrical optics, the smaller the diameter of the pinhole, the more favorable it is for improving examination accuracy; however, because the pinhole visual acuity measurement is mainly based on optical principles such as the light-converging effect of the pinhole, this examination method may be affected by diffraction in physical optics, making it difficult to measure the true potential best corrected visual acuity.

Nowadays, people view near objects more often than far objects. In some cases, accommodation is required to see near objects clearly. Improper eye accommodation may lead to blurred vision, visual fatigue, and may also cause the occurrence of myopia or further deterioration of myopia. The accommodative amplitude is an important factor in measuring the accommodative function. Currently, methods for measuring the accommodative amplitude are relatively rough and less popular. For example, some subjective measurement methods, such as the move-near method, the move-far method, and the negative lens method, mainly rely on the subjective judgment and feedback of the examinee to determine the measurement result, which is difficult to verify; meanwhile, during the measurement, the visual angle corresponding to the target keeps changing, so the examinee's accommodation cannot be precisely stimulated. The measurement result of the move-near method or the move-far method is usually obtained from the reciprocal of a working distance measured with a meter ruler, while the negative lens method adds a measured negative lens degree to the reciprocal of the working distance, where the working distance is the distance between the examinee's eye and the target. However, the measurement accuracy of these subjective measurement methods needs to be improved. For example, an objective measurement method for the accommodative amplitude includes dynamic retinoscopy, which usually requires an experienced operator, and automatic optometers are generally used for laboratory research; thus, this method is seldom applied.

At least one embodiment of the present disclosure provides an ophthalmic optical instrument, including: a visual target element, a first lens, a second lens, and a third lens; the first lens is spaced apart from the visual target element, light emitted from the visual target element is incident on the first lens and exits therefrom as parallel light; the second lens is located on a side of the first lens away from the visual target element, the parallel light is converged to form an image after passing through the second lens; and the third lens is located on a side of the second lens away from the first lens, the ophthalmic optical instrument further includes a control element connected to the second lens and configured to control movement of the second lens along its principal optical axis, so that an image formed by the second lens moves to at least one side of the third lens.

In the ophthalmic optical instrument provided by the embodiments of the present disclosure, the second lens can move along its principal optical axis under the control of the control element, so that the image formed by the second lens can move accordingly; for example, the image formed by the second lens can move on both sides of the third lens, e.g., to a side of the third lens away from the second lens. Thus, the image formed by the second lens (which serves as an object for the third lens) has a large movement range, thereby increasing the examination range and expanding the examined population.

Hereinafter, the ophthalmic optical instrument and the optical measurement system provided by the embodiments of the present disclosure will be described with reference to the drawings.

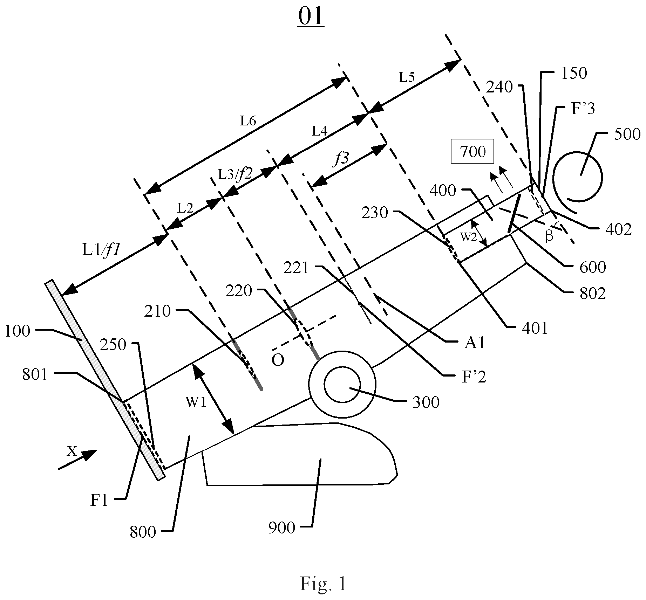

Fig. 1 is a schematic structural diagram of the ophthalmic optical instrument provided by at least one embodiment of the present disclosure.

As shown in Fig. 1, an ophthalmic optical instrument 01 includes a visual target element 100, a first lens 210, a second lens 220, and a third lens 230. The first lens 210 is spaced apart from the visual target element 100, and light emitted from the visual target element 100 enters the first lens 210 and exits therefrom as parallel light. The second lens 220 is located on a side of the first lens 210 away from the visual target element 100, and the parallel light is converged to form an image after passing through the second lens 220. The third lens 230 is located on a side of the second lens 220 away from the first lens 210. For example, in response to that the ophthalmic optical instrument is used to examine a human eye, the human eye is located on a side of the third lens away from the second lens.

In some embodiments, as shown in Fig. 1, the first lens 210 may be a collimator lens, and light emitted from the visual target element 100 exits as parallel light after passing through the first lens 210. For example, the second lens 220 includes a first positive lens; after the parallel light enters the second lens 220, an image plane 221 where the image formed by the second lens 220 is located is on a side of the second lens 220 away from the first lens 210 and at a back focal point F'2 of the second lens 220, and a distance between the image plane 221 and the second lens 220 is constant, i.e., equal to a focal length of the second lens 220. For example, the third lens 230 includes a second positive lens, and the image in the image plane 221 serves as an object for the third lens 230 and is imaged in an examinee's eye 500 after passing through the third lens 230, so as to stimulate the accommodation of the examinee's eye.

For example, as shown in Fig. 1, the ophthalmic optical instrument 01 further includes a control element 300 connected to the second lens 220 and configured to control the second lens 220 to move along a direction of its principal optical axis O, so that the image formed by the second lens 220 moves to at least one side of the third lens 230, thereby giving the examinee's eye different accommodative stimuli. For example, the direction of the principal optical axis O of the second lens 220 may be parallel to an X direction shown in Fig. 1. For example, the control element 300 may be directly connected to the second lens 220 or connected through a connection structure; the embodiments of the present disclosure do not limit the connection manner between the control element 300 and the second lens 220, as long as the control element 300 can control the movement of the second lens 220. In some embodiments, the control element 300 may include a rotary wheel, and an operator may rotate the rotary wheel to control the movement of the second lens 220. In some embodiments, the control element 300 may include a motor, and the operator may control the operation of the motor to move the second lens 220.

As shown in Fig. 1, that the image formed by the second lens 220 moves to at least one side of the third lens 230 means: along the direction of the principal optical axis O of the second lens 220, the image formed by the second lens 220 moves to a side of the third lens 230 close to the second lens 220, or to a side of the third lens 230 away from the second lens 220. For example, in response to that the second lens 220 moves away from the first lens 210, the image formed by the second lens 220 moves accordingly; for example, the image formed by the second lens 220 may be located on a side of the third lens 230 away from the second lens 220, and is then imaged on the examinee's eye 500 through the third lens 230 to examine the visual acuity of the examinee's eye 500. For example, in response to that the third lens 230 forms a clear image on the retina of the examinee, the best visual acuity of the examinee can be measured. In response to that the second lens 220 moves away from the examinee's eye, the second lens 220 moves to a critical position where the examinee's eye 500 can just distinguish between a clear image and an unclear image, which corresponds to a far point of accommodation; in response to that the second lens 220 moves toward the examinee's eye, the second lens 220 moves to a critical position where the examinee's eye 500 can just distinguish between a clear image and an unclear image, which corresponds to a near point of accommodation. A difference between a refractive power of the examinee corresponding to the far point of accommodation and a refractive power corresponding to the near point of accommodation represents the accommodative amplitude of the examinee.

As described above, in contrast to a general visual target element in which a target can only be located on a side of the third lens close to the visual target element, in the ophthalmic optical instrument provided by the present disclosure, the image of the target formed by the second lens may include two cases where the image is located on either side of the third lens; in particular, in response to that the image of the target is located on a side of the third lens away from the second lens, the accommodative range of the image of the target can be significantly increased, thereby improving the examination range of the ophthalmic optical instrument so as to adapt to a larger examined population.

For example, as shown in Fig. 1, the control element 300 is configured to control the second lens 220 to move between the first lens 210 and the third lens 230, and an image plane 221 of the second lens 220 is located on a side of the second lens 220 away from the visual target element 100. For example, the second lens 220 may move toward the first lens 210; the embodiments of the present disclosure do not limit a minimum distance between the first lens 210 and the second lens 220, as long as the parallel light exiting the first lens 210 can be incident on the second lens 220 and the second lens 220 can form an image. For example, the second lens 220 may also move toward the third lens 230, e.g., may move to be in contact with the third lens 230, in which case the image formed by the second lens 220 is located on a side of the third lens 230 away from the second lens 220. For example, the image formed by the second lens 220 may serve as an object for the third lens 230, and is not affected by the position and thickness (e.g., a dimension of the third lens 230 along its principal optical axis) of the third lens 230; it may be located on a side of the third lens 230 close to the second lens 220 or on a side of the third lens 230 away from the second lens 220. Therefore, the image formed by the second lens 220 has a large movement range, thereby improving the examination range. At the same time, compared with some solutions that directly move the target for examination, the embodiment of the present disclosure performs measurement by moving the image formed by the second lens 220, which also makes the operation easier and more stable.

As shown in Fig. 1, a front focal point F1 of the first lens 210 is on a side thereof away from the examinee's eye 500, and a back focal point thereof is on a side close to the examinee's eye 500. A front focal point of the second lens 220 is on a side away from the examinee's eye 500, and a back focal point thereof is on a side close to the examinee's eye 500. A front focal point of the third lens 230 is on a side away from the examinee's eye 500, and a back focal point F'3 thereof is on a side close to the examinee's eye. In some embodiments, a cylindrical lens may further be disposed at the back focal point F'3 of the third lens 230 to correct astigmatism, so as to measure a best corrected visual acuity achievable from refractive error to full correction. For example, during a follow-up measurement of the examinee, in response to that the measured best visual acuity decreases in a second measurement, but the same best visual acuity can be measured again after changing the position of the second lens 220 (thereby changing the position of the image formed by the second lens 220), then the decrease in best visual acuity in the second measurement may be caused by a change in refractive degree due to defocus of the refractive system of the examinee's eye. For example, in response to that the measured best visual acuity decreases in the second measurement, but the same best visual acuity cannot be measured after changing the position of the second lens 220 (thereby changing the position of the image formed by the second lens 220), then the decrease in the examinee's best visual acuity may be caused by an ocular organic disease.

For example, as shown in Fig. 1, a preset position A1 is defined between the first lens 210 and the third lens 230, and in response to that the second lens 220 moves between the preset position A1 and the third lens 230, an image plane 221 of the second lens 220 is located on a side of the third lens 230 away from the visual target element 100. For example, the preset position A1 is located between the first lens 210 and the third lens 230. Because the image formed by the second lens 220 is located at the back focal point of the second lens 220, in response to that a distance between the second lens 220 and the third lens 230 is smaller than a focal length of the second lens 220, the image formed by the second lens 220 is located on a side of the third lens 230 away from the second lens 220. Thus, a distance between the preset position A1 and the third lens 230 may be equal to (or smaller than) the focal length of the second lens 220.

For example, as shown in Fig. 1, a focal length f3 of the third lens 230 is greater than a focal length f2 of the second lens 220, i.e., in response to that the second lens 220 moves close to the third lens 230, the image formed by the second lens 220 can be located within the focal length range of the third lens 230. For example, in response to that the second lens 220 is located between the preset position A1 and the third lens 230, the image formed by the second lens 220 is located on a side of the third lens 230 away from the second lens 220; at this time, the image formed by the second lens 220 serves as a virtual object for the third lens 230, and the virtual object is located within the focal length range of the third lens 230, so that a real image can be formed by the third lens 230, allowing the examinee's eye 500 to see the real image. In the embodiments of the present disclosure, that an object is located within the focal length range of a lens means that a distance between the object and the lens is not greater than a focal length of the lens.

For example, as shown in Fig. 1, an entrance pupil center of the examinee's eye 500 coincides with the back focal point F'3 of the third lens 230. Similar to a telecentric optical measurement system, the examinee's pupil serves as an aperture stop; during movement of the second lens 220 along its principal optical axis O, the size of the final image formed on the examinee's retina remains unchanged. Compared with some optical measurement systems, for example, a Badal optometer whose back focal point is located at a front focal point of the examinee's eye 500, and a Nagel optometer whose back focal point is located at a nodal point of the examinee's eye 500, the premise for these two examination systems to keep the size of the image formed by the lens on the examinee's retina unchanged is that the examinee's retina must be clear, which has certain limitations and is difficult to fully guarantee in actual measurement. Therefore, in the ophthalmic optical instrument provided by the embodiments of the present disclosure, because the back focal point F'3 of the third lens 230 is located at the entrance pupil center of the examinee, the size of the image formed on the examinee's retina can remain constant whether the examinee's retina is clear or blurred.

Meanwhile, as shown in Fig. 1, in response to that the entrance pupil center of the examinee's eye 500 coincides with the back focal point F'3 of the third lens 230, a visual angle of the image formed by the image formed by the second lens 220 after passing through the third lens 230 with respect to the examinee's eye 500 remains unchanged. Compared with some measurement methods, for example, in the move-near method, as the target moves close to the examinee's eye, the visual angle of the target with respect to the examinee's eye increases; in the negative lens method, as the negative lens degree increases, the visual angle of the target with respect to the examinee's eye decreases. Because the size of the target is a main factor affecting the measurement of accommodative amplitude, it is extremely important for measurement accuracy to keep the visual angle of the image of the target relative to the examinee's eye (which is slightly greater than the examinee's best visual acuity) unchanged during measurement.

For example, as shown in Fig. 1, the ophthalmic optical instrument 01 further includes a lens barrel 400, which includes a first end 401 and a second end 402 opposite with each other, the third lens 230 is connected to the first end 401, and a central axis of the lens barrel 400 coincides with the principal optical axis of the third lens 230, a distance between the first end 401 and the second end 402 is not greater than the focal length f3 of the third lens 230. For example, the third lens 230 may be fixedly connected to the first end 401 of the lens barrel 400, and an end face of the second end 402 of the lens barrel 400 may serve as a detection surface, which is perpendicular to an extension direction of the principal optical axis O of the third lens 230, with the back focal point F'3 of the third lens 230 located on the detection surface. For example, the detection surface may be a virtual surface rather than an actually existing plane in the lens barrel 400. For example, during examination, the lens barrel 400 can reduce the influence of gas, impurities, etc. on an examination optical path, so as to improve examination accuracy. For example, the distance between the first end 401 and the second end 402 of the lens barrel 400 may be equal to the focal length of the third lens 230, so that the entrance pupil center of the examinee's eye 500 can coincide with the back focal point F'3 of the third lens 230 and be located on the detection surface of the second end 402 of the lens barrel 400, thereby providing operational convenience. In some embodiments, the distance between the first end 401 and the second end 402 of the lens barrel 400 may be smaller than the focal length of the third lens 230; in this case, the detection surface for the examinee's eye 500 is located outside the lens barrel 400, and for example, a position mark may be provided outside the lens barrel 400, and during examination, the examinee's entrance pupil center only needs to be located at the mark position.

For example, as shown in Fig. 1, a distance L6 between the first lens 210 and the third lens 230 is greater than a sum of the focal length f2 of the second lens 220 and the focal length f3 of the third lens 230. For example, in response to examining a hyperopic patient, in response to that the second lens 220 moves away from the third lens 230, a far point of accommodation of the examinee's eye is located outside the focal length range of the third lens 230; thus, by making the distance L6 between the first lens 210 and the third lens 230 greater than the sum of the focal length f2 of the second lens 220 and the focal length f3 of the third lens 230, support can be provided for examination of hyperopic patients, so as to detect potential hyperopia of the examinee. For example, in response to that the examinee has potential hyperopia, more accommodation is required relative to an emmetropic eye or a corrected eye, and phenomena such as visual fatigue are likely to occur. For example, a difference between the above L6 and f2+f3 may be set according to design requirements, e.g., may be 30 mm to 90 mm, such as 40 mm, 50 mm, 60 mm, or 70 mm, thereby expanding examination capability for hyperopic patients.

For example, as shown in Fig. 1, the visual target element 100 is located at a front focal point F1 of the first lens 210. For example, the first lens 210 is a collimator lens, and light emitted from the visual target element 100 exits as parallel light after refraction by the first lens 210, so that an image of the visual target element 100 formed by the first lens 210 is located at infinity.

For example, as shown in Fig. 1, the focal length f1 of the first lens 210 is larger than the focal length f2 of the second lens 220. Because the second lens 220 is a first positive lens, that is, the refractive power of the first lens 210 can be smaller than that of the second lens 220, the size of the image formed by the second lens 220 can be made smaller than that of the target in the target element 100, so that the size of the image entering the examinee's eye 500 is appropriate, and the vision detection range of the examinee can be expanded. In some embodiments, the refractive power of the first lens 210 may be 5D to 15D, such as 8D, 10D, 12D, 13D or 15D. In some embodiments, the refractive power of the second lens 210 may be 10D to 30D, such as 12D, 15D, 18D, 20D, 25D, 28D or 30D. For example, the ratio of the refractive power of the first lens 210 to the refractive power of the second lens 210 may be 0.2 to 0.8, for example, 0.3, 0.5, 0.6 or 0.8. For example, the focal length f2 of the second lens 220 may be 1/5 to 4/5 of the focal length f1 of the first lens 210, such as 1/4, 1/3 or 1/2, which is not limited by the embodiment of the present disclosure. For example, in response to that the refractive power of the first lens 210 is 1/2 of that of the second lens 220, the image formed by the visual target element 100 through the second lens 220 will be reduced to 1/2.

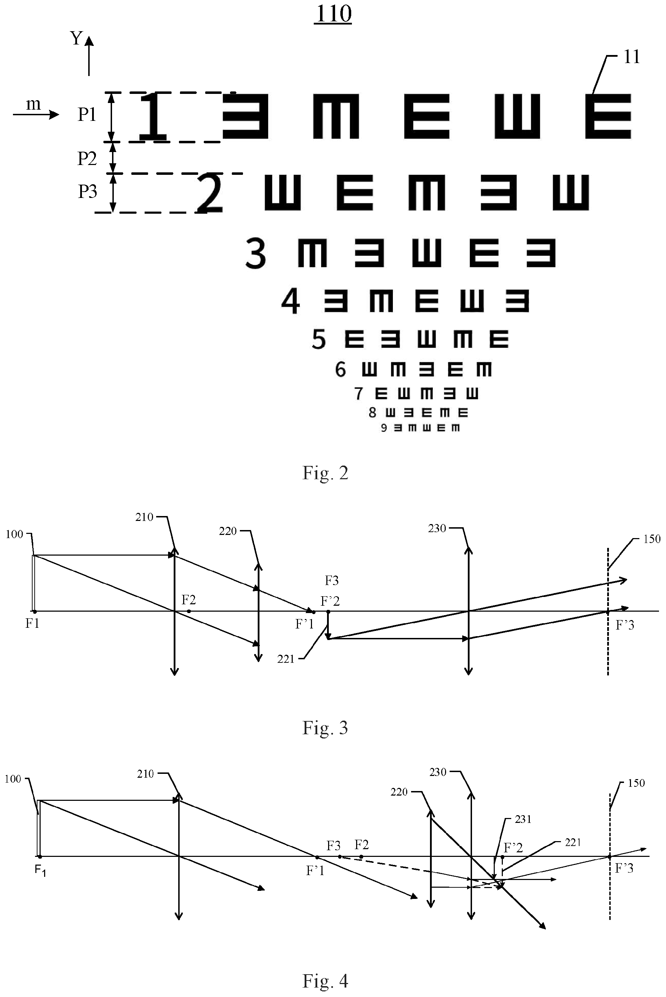

For example, as shown in Fig. 1, the visual target element 100 may include an electronic display panel, which is configured to display a visual target 110 (see Fig. 2). For example, the visual target element 100 may further include a controller to control the electronic display panel, so as to make it more intelligent. For example, in response to that the electronic display panel displays the visual target 110, the electronic display panel can adjust a display screen in a timely manner, e.g., can adjust display brightness for different application environments and different examinees, thereby enabling more flexible display. In addition, unlike some printed matters used for visual acuity examination, an intensity distribution of a display screen of the electronic display panel is a line spread function, such as an electronic screen seen in daily life, which is more consistent with the accommodative situation of modern people in response to using electronic products.

For example, as shown in Fig. 2, a shape and a size of the visual target 110 displayed on the electronic display panel are variable, so as to be selectable for clinical application and to prevent the examinee from memorizing the target, thereby verifying whether the examinee can really see the target clearly and improving examination accuracy.

In some embodiments, as shown in Fig. 1, in response to measuring the spectacle accommodation, the examinee's eye 500 wears corrected distance spectacle lens, and a surface of the corrected distance spectacle lens is placed against a measurement plane 150. A size of the selected target may be slightly larger than a size of the target corresponding to the examinee's best corrected visual acuity; then the second lens 220 is gradually moved away from the examinee, and the examinee is instructed to read the target in the visual target element 100 from clear to blurred, and continuously blurred, until the target in the visual target element 100 cannot be correctly read, at which point the target in the visual target element 100 is changed to prevent the examinee from memorizing it; then the second lens 220 is moved slightly closer until the examinee can correctly read the target, and at this time a position of the image plane of the second lens 220 is the far point of the examinee's eye. Then, the second lens 220 is gradually moved closer, and the examinee is instructed to read the target from clear to blurred and continuously blurred; the target in the visual target element 100 is changed to prevent the examinee from memorizing it; then the second lens 220 is moved slightly closer until the examinee can correctly read it, and at this time the position of the target is the near point of the examinee's eye 500. A difference between a refractive power of the examinee's eye 500 corresponding to the far point and a refractive power corresponding to the near point can be determined as the true accommodative amplitude of the examinee. For example, in a later follow-up examination of the examinee, in response to that the far point becomes closer, the examinee's eye 500 is myopized; in response to that the near point becomes farther, presbyopia is aggravated; and in response to that the far point and the near point approach each other, the accommodative amplitude decreases.

In some embodiments, as shown in Fig. 1 and Fig. 2, in response to measuring the ocular accommodation of an examinee, in response to that the examinee's eye 500 is emmetropic or wears contact lenses, the entrance pupil center of the examinee's eye 500 (i.e., at 3 mm behind the cornea) is located on the measurement plane 150, and the above measurement process is performed, so that the accommodative amplitude of the examinee's eye can be measured.

In some embodiments, as shown in Fig. 1, the electronic display panel is further configured to provide a voice prompt. For example, the electronic display panel can display a display screen that gradually moves closer (e.g., the target and/or images around the target gradually move closer); meanwhile, the electronic display panel can provide a voice prompt, e.g., can provide sound stimulation, to indicate a state such as that the target and the like are moving closer, thereby stimulating the examinee's proximal accommodation and measuring a maximum value.

Fig. 2 is a schematic diagram of a target provided by at least one embodiment of the present disclosure.

For example, as shown in Fig. 2, the visual target 110 may include at least one of a Chinese character, a numeral, and an English letter. For example, visual acuity measurement of an examinee may fall within the scope of psychophysics, which targets not only form sense but also perception. For example, the visual target 110 may include a combination of a Chinese character and an English letter, or a combination of a numeral and an English letter; the embodiments of the present disclosure do not limit this. For example, the visual target 110 may include English letters such as C, D, H, K, N, O, R, S, V, and Z, but is not limited thereto. For example, the visual target 110 may include numerals such as 2, 3, 4, 5, 6, 7, 8, and 9, but is not limited thereto. For example, the target shown in Fig. 2 includes a combination of a plurality of numerals and a plurality of English letters E, and the plurality of English letters E are arranged in different directions, e.g., different from a setting direction with respect to a Y direction shown in Fig. 2. The plurality of numerals are located on a same side of the plurality of English letters, but the embodiments of the present disclosure are not limited thereto. In some embodiments, the plurality of numerals and the plurality of English letters may be arranged alternately; for example, a row of numerals may be arranged between two adjacent rows of English letters, or a column of numerals may be arranged between two adjacent columns of English letters, so as to achieve a more diversified arrangement. In some embodiments, the visual target 110 may include a plurality of Chinese characters, and the plurality of Chinese characters may have different typefaces. For example, the plurality of English letters in the visual target 110 may also be different from each other, which helps improve examination accuracy. For example, the ophthalmic optical instrument provided by the embodiments of the present disclosure further includes an intelligent processor, which may be bound to an application (APP) to automatically judge an examinee's response result.

For example, as shown in Fig. 2, the visual target 110 includes a plurality of sub-visual targets 11, and the sub-visual targets 11 are arranged in M sub-visual target rows r, which are arranged along the Y direction shown in Fig. 2, and M is a positive integer greater than 1. For example, in the Y direction, the height P1 of the ith sub-target line R, the distance P2 between the ith sub-target line R and the (i+1)th sub-target line R, and the height P3 of the (i+1)th sub-target line R are distributed in geometric series, and i is a positive integer less than or equal to M-1. For example, the distance P2=P1/K1 and the distance P3=P1/K2. The above-mentioned geometric series distribution means that the values of P1 and P2 are geometrically distributed, and the values of P1 and P3 are geometrically distributed. For example, the distance P2=P1/101/20 (about P1/1.122) and the distance P3=P1/101/10 (about P1/1.2589), so that the sizes of the plurality of sub-targets R and the distances in the Y direction can be regularly distributed.

In some embodiments, as shown in Fig. 1, the ophthalmic optical instrument 01 can further measure an examinee's accommodation speed, i.e., a ratio of a time required to accommodate from the examinee's far point to the examinee's near point to the accommodative amplitude. In some embodiments, the ophthalmic optical instrument 01 can further measure an examinee's accommodation flexibility, i.e., a frequency of effectively changing accommodation between the examinee's far point and near point within a certain time; or a frequency of effectively changing accommodation between 5 meters and 0.3 meter within a certain time. In some embodiments, the ophthalmic optical instrument 01 can further measure an examinee's accommodation endurance, i.e., a duration for which the examinee's eye 500 can clearly see a visual target located at the near point. Certainly, the ophthalmic optical instrument 01 may have other measurement functions, and the embodiments of the present disclosure do not limit this.

For example, as shown in Fig. 1, the ophthalmic optical instrument 01 further includes a beam splitter 600 and an automatic detection element 700. The beam splitter 600 is located in the lens barrel 400, and a principal optical axis of the beam splitter 600 forms an inclination angle with a focal plane of the third lens 230. For example, in response to that the image formed by the third lens 230 is incident on the examinee's eye 500, part of the light is passed through the beam splitter 600 and part is reflected by the beam splitter 600; for example, one part of the light is incident on the examinee's eye 500 along the principal optical axis of the third lens 230 for subjective examination, and another part of the light may be reflected by the beam splitter 600 to the automatic detection element 700. For example, the automatic detection element 700 is configured to receive light from the beam splitter 600 so as to detect a diopter of the examinee's eye 500, thereby enabling objective measurement. For example, the automatic detection element 700 may be an automatic optometer, but is not limited thereto. With this arrangement, subjective examination and objective examination can be performed for the examinee's diopter; by comparing the subjective examination result and the objective examination result, an accommodative lead or accommodative lag in the subjective examination can be obtained, as well as a magnitude of the accommodative lead or a magnitude of the accommodative lag, so that a more reasonable vision accommodation plan can be formulated for the examinee based on the subjective and objective examination results.

In some embodiments, as shown in Fig. 1, the beam splitter 600 can be used to measure a pupil size of the examinee's eye, and a depth of field of the examinee's eye can be estimated by combining the pupil size of the examinee and the examinee's visual acuity examination result. For example, in other embodiments, the pupil size of the examinee's eye may also be directly measured through the beam splitter 600 to estimate the depth of field of the examinee's eye.

For example, as shown in Fig. 1, the inclination angle between the principal optical axis of the beam splitter 600 and the focal plane of the third lens 230 may be from 25 degrees to 50 degrees, such as 30 degrees, 35 degrees, 40 degrees, or 45 degrees, so that the light reflected and transmitted by the beam splitter 600 can be reasonably distributed, i.e., sufficient light can be emitted to the automatic detection element 700 while satisfying the subjective measurement.

For example, as shown in Fig. 1, the ophthalmic optical instrument 01 further includes a body 800 and a mounting frame 900. The body 800 is tubular, and the first lens 210, the second lens 220, and the third lens 230 are all located in the body 800. The body 800 includes opposite two ends (e.g., one end 801 and another end 802). Light emitted from the visual target element 100 enters the body 800 through the end 801 of the body 800 close to the first lens 210, and at least part of the lens barrel 400 extends into the body 800 from the other end 802 of the body 800. For example, the inner diameter of the first lens 210, the inner diameter of the second lens 220, and the inner diameter of the third lens 230 are all smaller than an inner diameter of the body 800, so that the body can provide a sufficiently large light propagation space. For example, the first lens 210 may be fixedly connected to the body 800. The visual target element 100 may have a display part, which is located in a minimum overlapping region of the visual target element 100 and the lens barrel 400 in the X direction, so as to ensure that light emitted from the visual target of the visual target element 100 can reach the first lens 210. For example, the lens barrel 400 may be fixedly connected to the body 800; part of the lens barrel 400 is located inside the body 800, and the third lens 230 is connected to the first end 401 of the lens barrel 400 to facilitate receiving light from the image formed by the second lens 220; another part of the lens barrel 400 is located outside the body 800 to match the examinee's eye 500 through its second end 402, so as to facilitate the examinee's eye 500 reaching a proper examination position.

For example, as shown in Fig. 1, the body 800 is rotatably connected to the mounting frame 900, so that an angle of the body 800 relative to a mounting plane of the mounting frame 900 is adjustable. For example, the mounting plane of the mounting frame 900 may be a horizontal plane, and a bottom surface of the mounting frame 900 is parallel to its mounting plane. A rotation axis is provided between the body 800 and the mounting frame 900, so that the body 800 can rotate about the rotation axis, thereby changing an angle of the body 800 relative to a design plane; for example, the angle may be from 25 degrees to 50 degrees, such as 30 degrees, 35 degrees, 40 degrees, or 45 degrees. For example, examinees differ in height, body shape, etc.; therefore, for different examinees, the angle of the body 800 relative to the design plane is different, e.g., the angle conforms to an angle at which the examinee views near objects. For example, for a shorter examinee, the angle may be appropriately reduced, so that the examinee can see the image of the visual target finally formed through the lenses along the direction X.

For example, as shown in Fig. 1, an outer diameter W2 of the lens barrel 400 is smaller than an inner diameter W1 of the body 800. For example, the outer diameter W2 of the lens barrel 400 may be 1/4 to 2/3 of the inner diameter W1 of the body 800, such as 1/3 or 1/2. With this arrangement, the examinee's eye can be easily aligned with the lens barrel 400 to achieve a good optical effect; the lens barrel 400 is light, easy to use, and its dimensional size can meet current clinical use requirements with high acceptability. For example, the lens barrel 400 further includes a fourth lens 240 at its second end 402, and the ophthalmic optical instrument 01 further includes a fifth lens 250 at the first end 801 of the body 800; for example, the fourth lens 240 and the fifth lens 250 may both be plane lenses, and the fourth lens 240 and the fifth lens 250 may be used to seal the ophthalmic optical instrument 01 for dust prevention.

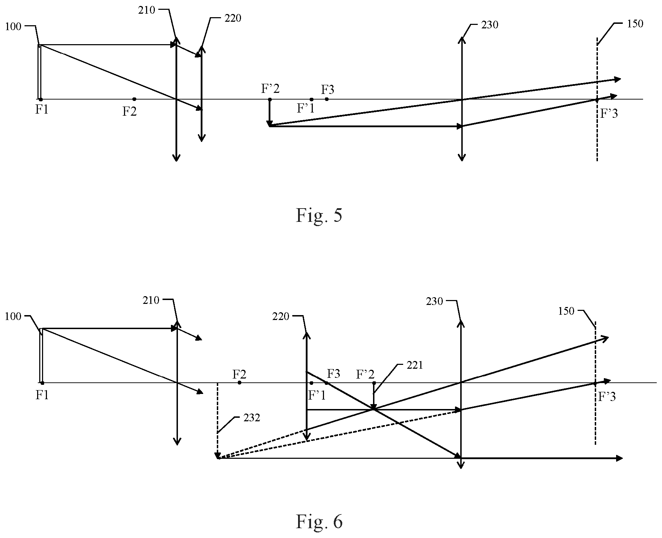

Fig. 3 is an optical path diagram in which the image formed by the second lens is located between the second lens and the third lens according to an embodiment of the present disclosure; Fig. 4 is an optical path diagram in which the image formed by the second lens is located on a side of the third lens away from the second lens according to an embodiment of the present disclosure; Fig. 5 is an optical path diagram of the ophthalmic optical instrument provided by an embodiment of the present disclosure in response to measuring a hyperopic eye; Fig. 6 is an optical path diagram of the ophthalmic optical instrument provided by an embodiment of the present disclosure in response to measuring a myopic eye. For example, Fig. 3 to Fig. 6 show a front focal point F1 and a back focal point F'1 of the first lens 210, a front focal point F2 and a back focal point F'2 of the second lens 220, and a front focal point F3 and a back focal point F'3 of the third lens 230. It should be noted that Fig. 3 to Fig. 6 only schematically show relative position relationships of the respective lenses and the visual target element, and the embodiments of the present disclosure do not limit the specific structures of the respective lenses and the visual target element.

For example, as shown in Fig. 3, the visual target element 100 serves as a real object; light emitted from the visual target element 100 is incident on the first lens 210 and imaged at infinity through the first lens 210. The parallel light emitted from the first lens 210 is incident on the second lens 220 and forms a real image at its back focal point F'2, and is located in an image plane 221. Because the image plane 221 is at the front focal point F3 of the third lens 230, the image formed by the second lens 220 serves as a real object for the third lens 230, and is thus imaged at infinity through the third lens 230. A back focal point F'3 of the third lens 230 is located in a measurement plane 150, and the examinee's eye coincides with the back focal point F'3 of the third lens 230; at this time, the examinee's eye is like viewing a far object, and an accommodative demand for the examinee's eye is 0. For example, in response to that the image formed by the second lens 220 gradually approaches the third lens 230, the image formed by the third lens 230 gradually approaches the examinee's eye, increasing the accommodative demand for the examinee's eye; at this time, the examinee needs to apply an equal amount of accommodation to see the image formed by the third lens 230 clearly.

For example, as shown in Fig. 4, a distance between the second lens 220 and the third lens 230 is smaller than a focal length of the second lens 220; at this time, the image formed by the second lens 220 is located on a side of the third lens 230 away from the second lens 220 and in the image plane 221. The image plane 221 serves as a virtual object for the third lens 230 and forms a real image 231 through the third lens 230; the real image 231 is located on a side of the third lens 230 away from the second lens 220 and between the image plane 221 and the third lens 230. The examinee's eye coincides with the back focal point F'3 of the third lens 230; at this time, the examinee's eye is like viewing a near object and needs accommodation to see it clearly. For example, a movement distance of the image formed by the second lens 220 has a linear relationship with the examinee's accommodative demand; in response to that the movement distance of the image formed by the second lens 220 decreases, the examinee's accommodative demand increases, thereby easily ensuring accuracy of the examination result.

For example, as shown in Fig. 5, the visual target element 100 serves as a real object; light emitted from the visual target element 100 is incident on the first lens 210 and imaged at infinity through the first lens 210. The parallel light emitted from the first lens 210 is incident on the second lens 220 and forms a real image at its back focal point F'2, i.e., in the image plane 221. The image formed by the second lens 220 serves as a real object for the third lens 230; because this real object is outside the front focal point F3 of the third lens 230 (i.e., on a side of the front focal point F3 of the third lens 230 close to the second lens 220), the image formed through the third lens 230 is located behind the retina of the examinee's eye and is imaged on the retina of the hyperopic patient through the refractive system of the hyperopic eye.

For example, as shown in Fig. 6, the visual target element 100 serves as a real object; light emitted from the visual target element 100 is incident on the first lens 210 and imaged at infinity through the first lens 210. The parallel light emitted from the first lens 210 is incident on the second lens 220 and forms a real image at its back focal point F'2, i.e., in the image plane 221. The image formed by the second lens 220 serves as a real object for the third lens 230; because this real object is within the front focal point F3 of the third lens 230 (i.e., on a side of the front focal point F3 of the third lens 230 close to the third lens 230), a virtual image 232 formed through the third lens 230 is located in front of the retina of the examinee's eye, and the examinee's eye at the measurement plane 150 is like viewing a near object.

At least one embodiment of the present disclosure further provides an optical measurement system, which includes the ophthalmic optical instrument according to any one of the foregoing embodiments. Because the optical measurement system includes the ophthalmic optical instrument according to any one of the foregoing embodiments, it also achieves the technical effects brought by the ophthalmic optical instrument, which will not be described herein again.

For example, the optical measurement system provided by at least one embodiment of the present disclosure may further include some control components, e.g., a remote controller for controlling display of the visual target element. The remote controller can be used to control the visual target element to display the multi-row sub-visual targets 11 as shown in Fig. 2, or to control the visual target element 110 to display a certain row of targets alone, or to control the visual target element 110 to display a single sub-target 11 alone. For example, the remote controller may further control a background color of the visual target element 110 to change; for example, it may make a background color of part of the sub-targets 11 red, a background color of part of the sub-targets 11 green, and a background color of part of the sub-targets 11 white, so as to judge whether the examinee's current refractive correction is over-correction or under-correction. For example, the remote controller may further control colors of the sub-targets 11 shown in Fig. 2 to be swapped with colors of their backgrounds; for example, it may make the sub-targets 11 in Fig. 2 white and their backgrounds black, so as to judge whether the examinee has visual acuity degradation caused by a turbid refractive medium, such as a cataract. For example, the remote controller may further control the visual target element 110 to display an astigmatic dial, so as to judge whether astigmatism exists. The embodiments of the present disclosure do not limit the control functions of the remote controller.

For example, in the optical measurement system provided by at least one embodiment of the present disclosure, the control component may further include a controller connected to the automatic optometer, so as to control inputting and recording of an examinee's name, time, number, etc., and to enable one-key automatic optometry and saving, so as to record a result of each follow-up of the subject and display it as a refraction curve, which can be compared with a subjective measurement value. The embodiments of the present disclosure do not limit the functions of the controller.

The following points also need to be noted:
(1) In the drawings of the embodiments of the present disclosure, only structures involved in the embodiments of the present disclosure are shown; other structures may refer to conventional designs.
(2) Features in the same embodiment and in different embodiments of the present disclosure may be combined with each other if there is no conflict.

The foregoing is merely an exemplary embodiment of the present disclosure, rather than limiting the protection scope of the present disclosure, which is defined by the appended claims.

## Claims

1. An ophthalmic optical instrument, comprising:
a visual target element;
a first lens, spaced apart from the visual target element, wherein light emitted from the visual target element enters the first lens and exits as parallel light;
a second lens, located on a side of the first lens away from the visual target element, wherein the parallel light is converged to form an image after passing through the second lens; and
a third lens, located on a side of the second lens away from the first lens,
wherein the ophthalmic optical instrument further comprises a control element, the control element is connected to the second lens and configured to control movement of the second lens along its principal optical axis, so that an image formed by the second lens moves to at least one side of the third lens.

2. The ophthalmic optical instrument according to claim 1, wherein the first lens is a collimator lens, the second lens comprises a first positive lens, and the third lens comprises a second positive lens,
wherein the control element is configured to control the second lens to move between the first lens and the third lens, and an image plane of the second lens is located on a side of the second lens away from the visual target element.

3. The ophthalmic optical instrument according to claim 2, wherein a preset position is defined between the first lens and the third lens,
wherein in response to that the second lens moves between the preset position and the third lens, the image plane of the second lens is located on a side of the third lens away from the visual target element.

4. The ophthalmic optical instrument according to claim 3, wherein a focal length of the third lens is greater than a focal length of the second lens.

5. The ophthalmic optical instrument according to any one of claims 2 to 4, further comprising a lens barrel, wherein the lens barrel comprises a first end and a second end opposite to each other, the third lens is connected to the first end, and a central axis of the lens barrel coincides with a principal optical axis of the third lens, and a distance between the first end and the second end is not greater than a focal length of the third lens.

6. The ophthalmic optical instrument according to claim 5, wherein an entrance pupil center of an examinee's eye coincides with a back focal point of the third lens.

7. The ophthalmic optical instrument according to any one of claims 1 to 6, wherein a focal length of the first lens is greater than a focal length of the second lens.

8. The ophthalmic optical instrument according to claim 7, wherein the focal length of the second lens is in a range from 1/5 to 4/5 of the focal length of the first lens.

9. The ophthalmic optical instrument according to any one of claims 1 to 8, wherein a distance between the first lens and the third lens is greater than a sum of a focal length of the second lens and a focal length of the third lens.

10. The ophthalmic optical instrument according to any one of claims 1 to 9, wherein the visual target element is located at a front focal point of the first lens.

11. The ophthalmic optical instrument according to claim 6, further comprising:
a beam splitter, located in the lens barrel, wherein a principal optical axis of the beam splitter forms an inclination angle with a focal plane of the third lens, the inclination angle is in a range from 25 degrees to 50 degrees; and
an automatic detection element, configured to receive light from the beam splitter so as to detect a diopter of the examinee's eye.

12. The ophthalmic optical instrument according to any one of claims 1 to 11, wherein the visual target element comprises an electronic display panel configured to display a visual target,
wherein the visual target comprises at least one of a Chinese character, a numeral, and an English letter, and the Chinese character comprises at least one typeface.

13. The ophthalmic optical instrument according to claim 12, wherein a shape and a size of the visual target are variable.

14. The ophthalmic optical instrument according to claim 12 or 13, wherein the electronic display panel is further configured to provide a voice prompt.

15. The ophthalmic optical instrument according to any one of claims 12 to 14, wherein the visual target comprises a plurality of sub-targets arranged as M sub-target rows, where M is a positive integer greater than 1,
wherein along an arrangement direction of the M sub-target rows, a height of an i-th sub-target row, a distance between the i-th sub-target row and an (i+1)-th sub-target row, and a height of the (i+1)-th sub-target row are distributed in a geometric progression, where i is a positive integer less than or equal to M-1.

16. The ophthalmic optical instrument according to claim 5 or 6, further comprising: a body and a mounting frame, wherein the body is tubular, the first lens, the second lens, and the third lens are all located in the body, the body comprises two ends opposite with each other, the light emitted from the visual target element enters the body through an end of the body close to the first lens, and at least part of the lens barrel extends into the body from another end of the body,
wherein the body is rotatably connected to the mounting frame, so that an angle of the body relative to a setup plane of the mounting frame is adjustable.

17. The ophthalmic optical instrument according to claim 16, wherein an outer diameter of the lens barrel is smaller than an inner diameter of the body.

18. An optical measurement system, comprising the ophthalmic optical instrument according to any one of claims 1 to 17.
